(19) **Europäisches Patentamt**

**European Patent Office**

**Office européen des brevets**

(11) **EP 1 110 933 B1**

(12) **FASCICULE DE BREVET EUROPEEN**

(45) Date de publication et mention
de la délivrance du brevet:
**31.03.2004 Bulletin 2004/14**

(51) Int Cl.[7]: **C07C 11/06**

(21) Numéro de dépôt: **00403480.7**

(22) Date de dépôt: **11.12.2000**

(54) **Procédé de production sélective de propylène à partir de coupes d'hydrocarbures à quatre atomes de carbone**

Verfahren zur selektiven Herstellung von Propen aus Kohlenwasserstoffschnitten mit vier Kohlenstoffatomen

Process for the selective production of propylene from hydrocarbon cuts with four carbon atoms

(84) Etats contractants désignés:
**DE ES FR GB IT NL**

(30) Priorité: **24.12.1999 FR 9916506**

(43) Date de publication de la demande:
**27.06.2001 Bulletin 2001/26**

(73) Titulaire: **Institut Français du Pétrole**
**92852 Rueil-Malmaison Cedex (FR)**

(72) Inventeurs:
• **Commereuc, Dominique**
**92190 Meudon (FR)**
• **Travers, Christine**
**92500 Rueil Malmaison (FR)**

(56) Documents cités:
**FR-A- 2 755 130**

**Description**

L'invention concerne un procédé de production sélective de propylène de qualité polymérisation à partir d'une coupe $C_4$ oléfinique.

Le vapocraquage de charges constituées par des coupes paraffiniques légères fournit l'éthylène et le propylène nécessaires à la pétrochimie. Il fournit également un certain nombre d'autres produits plus lourds, et en particulier une coupe d'hydrocarbures en $C_4$ qui contient principalement du butadiène-1,3, de l'isobutène, des n-butènes et des butanes, accompagnés de traces d'hydrocarbures acétyléniques.

Le craquage catalytique de charges d'hydrocarbures lourds fournit, à côté des fractions essence et gazole qui sont les produits principaux, des produits plus légers, parmi lesquels une coupe d'hydrocarbures en $C_4$ qui contient principalement de l'isobutane, de l'isobutène, des n-butènes et des butanes, accompagnés de faibles quantités de butadiène-1,3 et d'hydrocarbures acétyléniques.

Jusqu'à récemment, seuls le butadiène-1,3 et l'isobutène trouvaient un usage dans l'industrie des polymères, en particulier dans l'industrie des pneumatiques. L'augmentation de la longévité des pneumatiques et une relative stagnation de la demande font qu'il existe maintenant des surplus de butadiène qui ne sont pas, ou mal, utilisés. L'isobutène était jusqu'à présent utilisé par exemple pour la synthèse d'éthers à usage d'additifs dans les carburants automobiles ou comme monomère dans la synthèse de polyisobutène. Cependant ces usages peuvent arriver à saturation et laisser l'isobutène sans utilisation.

La présente invention propose un procédé de traitement d'une coupe d'hydrocarbures en $C_4$ contenant principalement de l'isobutène, des n-butènes, des butanes, et du butadiène-1,3 en quantité variable, qui inclut l'isomérisation squelettale de l'isobutène en n-butènes, et qui permet de transformer l'ensemble des composés insaturés en $C_4$ en propylène utilisable par exemple pour la polymérisation.

Les coupes traitées dans le procédé selon l'invention correspondent aux coupes $C_4$ de procédés de conversion. Elles peuvent par exemple correspondre à la coupe $C_4$ brute de vapocraquage, à la coupe $C_4$ de vapocraquage après extraction du butadiène communément appelée raffinat-1, ou à la coupe $C_4$ de craquage catalytique.

Les proportions relatives d'éthylène et de propylène produits dans une opération de vapocraquage peuvent être modulées dans une certaine mesure en changeant la nature de la charge et en modifiant les conditions opératoires (la sévérité) du craquage. Cependant, le mode de fonctionnement orienté vers une proportion plus grande de propylène entraîne inévitablement une baisse du rendement en éthylène et une production plus importante de coupe $C_4$ et de fraction essence.

Un autre but de la présente invention est ainsi d'augmenter la production de propylène tout en maintenant un haut rendement en éthylène grâce au traitement de la coupe d'hydrocarbures en $C_4$ et donc sans que l'on soit obligé de baisser la sévérité du vapocraqueur.

Le procédé objet de l'invention est plus précisément un procédé de conversion en propylène d'une coupe $C_4$ oléfinique, ladite coupe comprenant des dioléfines, principalement du butadiène-1,3, du butène-1, du butène-2, de l'isobutène et des impuretés acétyléniques, ledit procédé comprenant les étapes suivantes, se déroulant successivement :

1) l'hydrogénation sélective des dioléfines et des impuretés acétyléniques avec isomérisation du butène-1 en butènes-2, menée dans un réacteur, en présence d'un catalyseur, de façon à obtenir un effluent contenant majoritairement des butènes-2 et de l'isobutène, et ne contenant pratiquement pas de dioléfines ni de composés acétyléniques ;

2) la séparation par distillation d'une coupe de tête contenant majoritairement l'isobutène et le butène-1 non converti dans la première étape, et d'une coupe de pied contenant essentiellement le butène-2 et le butane ; et

4) la métathèse de la coupe butènes-2 issue de l'étape 2 avec de l'éthylène façon à obtenir un effluent contenant du propylène, la métathèse étant suivie d'une séparation du propylène ;

ledit procédé comprenant en outre une étape 3 d'isomérisation squelettale de l'isobutène en n-butènes sur la fraction de tête de l'étape 2 de distillation, avec recyclage d'au moins une partie de l'effluent à l'étape 1, ladite étape 3 étant réalisée au moyen d'un catalyseur comprenant de l'alumine et du titane à une température de 300 °C à 570 °C, une pression de 0,1 à 1 MPa, à une vitesse spatiale de 0,1 à 10 h$^{-1}$, et en présence d'injection d'eau, le rapport molaire eau injectée / hydrocarbures oléfiniques étant de 0,1 à 10.

L'isomérisation du butène-1 en butènes-2 telle que réalisée dans l'étape 1 peut aussi en partie être réalisée en association avec la distillation (étape 2) par mise en oeuvre d'un catalyseur d'isomérisation tel que décrit pour l'étape 1 selon les enseignements du français FR-B-2 755 130, au nom de la demanderesse.

On décrit ci-après plus en détail les conditions particulières des différentes étapes du procédé selon l'invention, réalisé à partir d'une coupe d'hydrocarbures en $C_4$ contenant principalement de l'isobutène, des n-butènes, des butanes, ainsi que du butadiène en quantité variable, ladite coupe $C_4$ étant soumise à ces étapes pour produire es-

2

sentiellement du propylène.

L'objet principal de la première étape est de transformer le butadiène et les n-butènes en butènes-2. En effet, les butènes-2 sont la source du propylène qui est produit dans l'étape 4 de métathèse en présence d'éthylène. Il est donc souhaitable de maximiser le rendement en butènes-2, c'est-à-dire de se rapprocher autant que possible de la proportion autorisée par la thermodynamique. Le deuxième objet de cette étape est d'éliminer les traces d'hydrocarbures acétyléniques toujours présentes dans ces coupes et qui sont des poisons ou des polluants pour les étapes ultérieures.

Dans cette première étape, on réalise donc simultanément les réactions suivantes, en présence d'hydrogène :

- l'hydrogénation sélective du butadiène en un mélange de n-butènes ;
- l'isomérisation du butène-1 en butènes-2, de manière à obtenir une répartition proche de l'équilibre thermodynamique ; et
- l'hydrogénation sélective des traces d'hydrocarbures acétyléniques en butènes.

Ces réactions peuvent être réalisées au moyen de divers catalyseurs spécifiques comprenant un ou plusieurs métaux, par exemple du groupe 10 de la classification périodique (Ni, Pd ou Pt), déposés sur un support. On met en oeuvre de préférence un catalyseur qui comprend au moins un composé de palladium fixé sur un support minéral réfractaire, par exemple sur une alumine. La teneur en palladium sur le support peut être de 0,01 à 5 % en poids, de préférence de 0,05 à 1 % en poids. Divers modes de pré-traitement connus de l'homme du métier peuvent éventuellement être appliqués à ces catalyseurs pour en améliorer la sélectivité dans l'hydrogénation du butadiène en butènes aux dépens de l'hydrogénation totale en butane, qu'il faut éviter. De préférence, le catalyseur contient de 0,05 à 10 % en poids de soufre. De façon avantageuse, on utilise un catalyseur comprenant du palladium déposé sur de l'alumine, et du soufre.

Le catalyseur peut avantageusement être mis en oeuvre selon le procédé décrit dans le brevet FR-B-2 708 596. Selon ce procédé, le catalyseur est traité, avant son chargement dans le réacteur d'hydrogénation, par au moins un composé soufré dilué dans un solvant, puis le catalyseur obtenu contenant 0,05 à 10 % en poids de soufre est chargé dans le réacteur et activé sous atmosphère neutre ou réductrice à une température de 20 à 300 °C, une pression de 0,1 à 5 MPa et une VVH de 50 à 600 h$^{-1}$, et la charge est mise au contact dudit catalyseur activé.

La mise en oeuvre du catalyseur, de préférence au palladium, n'est pas critique, mais on préfère généralement utiliser au moins un réacteur à flux descendant à travers un lit fixe de catalyseur. Lorsque la proportion de butadiène dans la coupe est importante, ce qui est le cas par exemple d'une coupe de vapocraquage lorsqu'on ne souhaite pas en extraire le butadiène pour des usages spécifiques, il peut être avantageux d'effectuer la transformation dans deux réacteurs en série afin de mieux contrôler la sélectivité de l'hydrogénation. Le second réacteur peut être à flux ascendant et avoir un rôle de finition.

Dans certains cas, il peut être judicieux de diluer la charge à traiter par ladite coupe C$_4$ dans laquelle le butadiène est partiellement ou totalement hydrogéné.

La quantité d'hydrogène nécessaire à l'ensemble des réactions réalisées dans cette étape est ajustée en fonction de la composition de la coupe pour avoir avantageusement seulement un léger excès d'hydrogène par rapport à la stoechiométrie.

Les conditions opératoires sont choisies de telle façon que les réactifs et les produits soient en phase liquide, et qu'elles favorisent la formation des butènes-2. Il peut cependant être avantageux de choisir un mode de fonctionnement tel que les produits soient partiellement vaporisés à la sortie du réacteur, ce qui facilite le contrôle thermique de la réaction. La température peut varier de 0 à 200 °C, de préférence de 0 à 150 °C ou mieux de 0 à 70 °C. La pression peut être ajustée à une valeur de 0,1 à 5 MPa, de préférence de 0,5 à 4 MPa et avantageusement de 0,5 à 3 MPa, de telle façon que les réactifs, au moins en partie, soient en phase liquide. La vitesse spatiale peut être de 0,5 à 20 h$^{-1}$ et de préférence de 1 à 10 h$^{-1}$, avec un rapport molaire H$_2$ / dioléfines de 0,5 à 5 et de préférence de 1 à 3.

Le ou les réacteurs d'hydro-isomérisation peuvent avantageusement être suivis d'une colonne de stabilisation qui élimine les traces d'hydrocarbures gazeux éventuellement présents dans l'hydrogène de charge.

L'objet de la deuxième étape est de séparer par distillation la coupe C$_4$ issue de l'étape précédente, pour obtenir d'une part une fraction contenant l'isobutène, l'isobutane et la majorité du butène-1, d'autre part une fraction contenant une faible quantité de butène-1, les butènes-2 et le n-butane L'isobutène ainsi concentré est conduit à l'étape 3 d'isomérisation squelettale. La fraction butènes-2 est dirigée vers l'étape de métathèse.

Afin de réduire le plus possible la concentration en butène-1 dans l'effluent de la tête de colonne, il est possible de mettre en oeuvre une colonne de distillation réactive comportant, intérieurement à la colonne ou extérieurement, une ou plusieurs charges du catalyseur mis en oeuvre comme décrit pour l'étape 1. La colonne de distillation réactive employée peut alors être de n'importe quel type. Dans une disposition préférée, au moins une zone contenant le catalyseur est aménagée. La disposition mécanique du catalyseur dans la ou les zones catalytiques doit être telle qu'elle gêne le moins possible les flux de vapeur et de liquide entre les deux zones de séparation qui l'encadrent. Le catalyseur peut par exemple être disposé en couche mince sur des plateaux perforés ou sur des grilles, ou dans des sachets suspendus ou posés sur des supports assurant leur tenue mécanique, ou de toute autre façon connue de l'homme du métier. D'autre part, le catalyseur peut être disposé dans la colonne de manière à n'être traversé que par un flux ascendant de phase liquide. Il peut également être disposé sous forme de garnissage catalytique, suivant les différents modes de mise en oeuvre connus. Les zones de séparation qui encadrent les zones catalytiques peuvent comporter des plateaux ou du garnissage.

Une des mises en oeuvre de la colonne peut par exemple correspondre à celle décrite dans le brevet français FR-B-2 755 130 au nom de la demanderesse.

La fraction de tête de distillation, riche en isobutène, est soumise dans l'étape 3 à une isomérisation squelettale destinée à transformer l'isobutène en n-butènes, qui pourront être renvoyés à l'entrée de la zone 1. On pourra purger l'isobutane éventuellement présent.

Cette réaction d'isomérisation squelettale peut être réalisée grâce à des catalyseurs à base d'alumine ou plus particulièrement d'alumines activées ou traitées à la vapeur (brevet US-A-3 558 733) ou comprenant des composés comme ceux du titane (brevet US-A-5 321 195 de la demanderesse) et/ou du bore (brevet US-A-5 659 104 de la demanderesse) qu'il s'agisse d'alumines éta ou gamma, d'alumines halogénées (brevet US-A-2 417 647) ou de bauxite. Des zéolithes ou tamis moléculaires possédant un réseau microporeux mono-dimensionnel (documents de brevet EP-A-523 838, EP-A-501 577 et EP-A-740 957 de la demanderesse) peuvent également constituer des phases actives de catalyseurs d'isomérisation squelettale. Les catalyseurs à base d'alumine sont généralement mis en oeuvre en présence d'eau à des températures allant de 200 °C à 700 °C, à une pression de 0,1 à 2 MPa, à une vitesse spatiale de 0,1 à 20 h$^{-1}$ et avec un rapport molaire eau injectée sur hydrocarbure de 0,1 à 10. Les catalyseurs zéolithiques sont mis en oeuvre en l'absence d'eau, à une température de 200 °C à 500 °C, sous une pression de 0,1 à 2 MPa et à une vitesse spatiale de 0,1 à 20 h-1.

On utilisera de préférence dans l'invention un catalyseur contenant de l'alumine et de 0,03 à 0,6 % en poids de titane et pouvant renfermer également de 0,05 à 5 % en poids d'un oxyde d'un élément du groupe IIIA, cet élément étant avantageusement le bore. Avant d'être mis au contact des hydrocarbures de la charge, ce catalyseur aura avantageusement subi un traitement à la vapeur d'eau, à une température de 120 à 700 °C sous une pression partielle de vapeur d'eau supérieure à 0,05 MPa, pendant une durée de 0,5 à 120 heures.

La fraction du fond de zone de distillation, riche en butènes-2, contient de préférence au plus 1 % en poids de butène-1, avantageusement au plus 0,5 % en poids, et au plus 1 % en poids d'isobutène. La fraction butènes-2 issue de l'étape 2 ne contient pas de polluant extérieur et peut donc être envoyée directement dans la quatrième étape du procédé. Dans cette dernière étape, les butènes-2 sont mis en réaction avec de l'éthylène, pour donner du propylène par métathèse En raison de la faible quantité de butène-1 dans la charge, la formation de sous-produits est très limitée.

La réaction de métathèse de l'éthylène avec les butènes-2 peut être catalysée par des oxydes métalliques variés déposés sur des supports, par exemple par des oxydes de molybdène, de tungstène ou de rhénium. On utilise de préférence un catalyseur comprenant au moins un oxyde de rhénium déposé sur un support comprenant un oxyde réfractaire contenant lui-même au moins de l'alumine, et qui présente un caractère acide, comme par exemple l'alumine elle-même, les silice-alumines ou les zéolithes.

On peut citer à titre d'exemples préférés les catalyseurs comprenant de l'heptoxyde de rhénium déposé sur une alumine gamma, tels que ceux décrits dans le brevet US-A-4 795 734. La teneur en rhénium (exprimée en rhénium métallique) peut être de 0,01 à 20 %, de préférence de 1 à 15 % en poids. Les catalyseurs sont soumis par exemple à une activation thermique finale à une température de 400 à 1000 °C pendant une durée de 10 minutes à 5 heures sous une atmosphère non réductrice.

Les catalyseurs comprenant de l'heptoxyde de rhénium déposé sur une alumine peuvent aussi être modifiés par l'adjonction d'un oxyde d'un autre métal. De tels catalyseurs modifiés comprennent par exemple du rhénium à l'état d'oxyde, à raison de 0,01 à 20 % en poids exprimé en rhénium métallique, déposé sur un support contenant au moins 75 % en poids d'alumine et de 0,01 à 30 % en poids d'au moins un oxyde d'un métal choisi dans le groupe formé par le niobium et le tantale, tel que décrit dans le brevet FR-B-2 709 125. Une autre classe de catalyseurs modifiés comprend du rhénium à l'état d'oxyde, à raison de 0,01 à 20 % en poids exprimé en rhénium métallique, déposé sur un support contenant au moins 75 % en poids d'alumine et de 0,01 à 10 % en poids en aluminium d'un composé de formule $(RO)_qAlR'_r$, où R est un reste hydrocarbyle de 1 à 40 atomes de carbone, R' est un reste alkyle de 1 à 20 atomes de carbone et q et r sont égaux à 1 ou 2, avec q + r égal à 3 (voir le brevet FR-B-2 740 056).

La réaction de métathèse est effectuée de préférence en phase liquide, en l'absence d'oxygène, de composés oxygénés et d'humidité, et à une température de 0 à 200 °C, de préférence de 20 à 150 °C, sous une pression au moins égale à la tension de vapeur du mélange réactionnel à la température de réaction.

Le catalyseur peut être mis en oeuvre en lit fixe. Cependant, comme il doit être fréquemment régénéré, il est alors nécessaire de disposer d'au moins deux réacteurs en parallèle, l'un étant en fonctionnement pendant que l'autre est en régénération. On utilise de préférence un système de lit catalytique mobile comme décrit dans le brevet français FR-B-2 608 595. Le catalyseur est soutiré à intervalles de temps réguliers en bas du réacteur et transféré vers un système de régénération en continu, d'où il est renvoyé en haut du réacteur.

Compte tenu des limitations imposées par la thermodynamique, l'éthylène non converti est fractionné dans une première colonne de distillation et recyclé au réacteur de métathèse. Une deuxième colonne de distillation sépare le propylène et les hydrocarbures en $C_4$ non convertis, qui peuvent être recyclés au réacteur de métathèse ou à un autre endroit du procédé.

Lorsque le procédé est appliqué à une coupe $C_4$ de vapocraquage, il peut être avantageux d'intégrer l'unité de métathèse avec le craqueur, de manière à bénéficier du train de fractionnement de celui-ci. On utilise alors dans l'étape de métathèse de l'éthylène provenant de l'opération de vapocraquage.

La succession des traitements retenue dans le procédé selon l'invention présente de nombreux avantages. Les composés les plus réactifs de la coupe, en particulier le butadiène-1,3 présent en quantités variables, ainsi que les traces d'hydrocarbures acétyléniques, sont transformés dès la première étape, et ne seront donc pas la cause de réactions parasites dans les étapes suivantes. Par ailleurs, l'hydrogénation sélective des dioléfines (butadiène-1,3 et le cas échéant butadiène-1,2) en butènes, l'hydro-isomérisation du butène-1 et l'isomérisation squelettale de l'isobutène en n-butènes permettent d'augmenter considérablement la concentration en butènes-2 dans la coupe, ce qui favorise d'autant un rendement élevé en propylène dans l'étape de métathèse.

Le fractionnement de la coupe issue de l'hydroisomérisation, en isobutène et butène-1 d'une part, et en butènes-2 d'autre part, permet de concentrer l'isobutène pour l'étape d'isomérisation squelettale, ainsi que les butènes-2 qui sont ensuite soumis à la métathèse.

De plus, dans l'étape suivante de métathèse (étape 4), la faible teneur en butène-1 dans la fraction riche en butènes-2 permet d'obtenir une sélectivité en propylène voisine de 100 %. On sait en effet que le butène-1 réagit avec les butènes-2 pour donner du propylène et des pentènes, et qu'il réagit sur lui-même pour donner des hexènes. Pentènes et hexènes sont des sous-produits de faible valeur, dont il faut se débarrasser, de manière coûteuse. Le procédé permet donc une augmentation appréciable du rendement en propylène, et facilite le recyclage des butènes-2 au réacteur de métathèse, puisqu'il y a peu de pentènes et d'hexènes à éliminer.

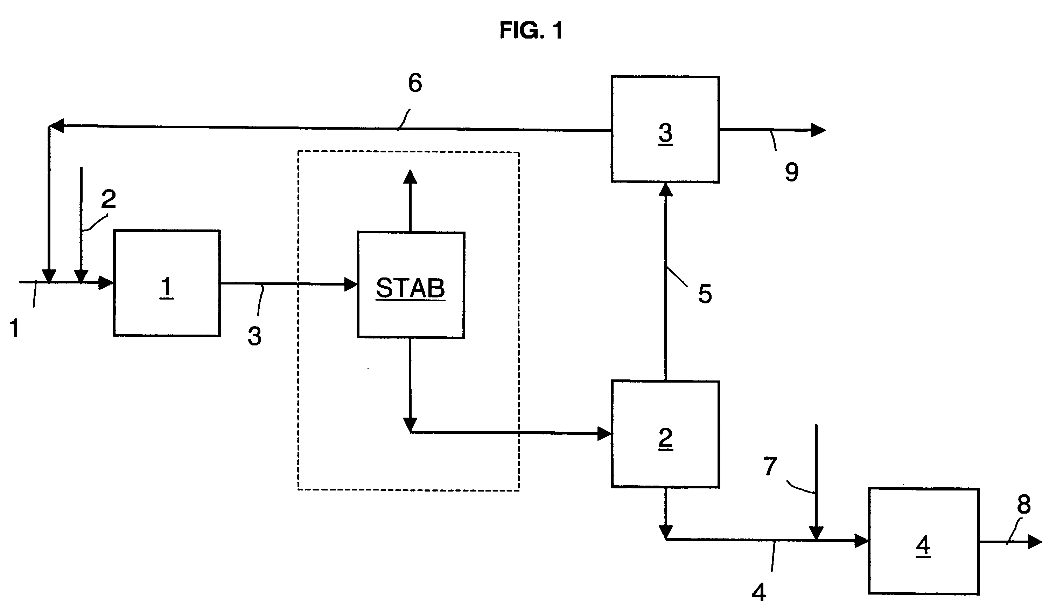

L'invention concerne également une installation (illustrée par la figure 1) utilisée pour mettre en oeuvre le procédé décrit ci-dessus.

Elle comporte successivement :

- une zone 1 d'hydrogénation sélective avec isomérisation du butène-1 en butène-2, ladite zone comportant au moins un moyen 1 pour l'introduction de la coupe C4 à convertir, au moins un moyen 3 pour la sortie de l'effluent et au moins un moyen 2 pour l'introduction de l'hydrogène, ladite zone comportant également au moins un lit de catalyseur ;
- une zone 2 de séparation, comportant au moins un moyen 3 pour l'introduction de l'effluent issu de la zone 1, au moins un moyen 5 pour la sortie de l'isobutène et du butène-1, au moins un moyen 4 pour la sortie du butène-2 et du n-butane ; et
- -une zone 4 de métathèse contenant au moins un lit de catalyseur et comprenant au moins un moyen 4 d'introduction de l'effluent issu de la zone 2, au moins un moyen 7 d'introduction de l'éthylène et au moins un moyen 8 pour la sortie du propylène.

ladite installation comprenant en outre une zone 3 d'isomérisation squelettale, comportant au moins un moyen 5 pour l'introduction de l'effluent issu de la zone 2, au moins un moyen 6 pour le recyclage de la sortie de la zone 3 à l'entrée de la zone 1 et au moins un moyen 9 pour purger l'isobutane éventuellement présent, ladite zone comportant également au moins un lit de catalyseur comprenant de préférence de l'alumine et du titane.

De façon particulièrement intéressante, la coupe $C_4$ provient d'une zone de vapocraquage en amont, le moyen d'introduction de la coupe à convertir dans la zone 1 étant relié à ladite zone de vapocraquage, et le moyen d'introduction de l'éthylène dans la zone 4 étant relié à ladite zone de vapocraquage.

L'exemple suivant illustre l'invention sans en limiter la portée.

**EXEMPLE**

Une coupe $C_4$ en sortie de vapocraqueur présente la composition indiquée dans le tableau 1 (flux 1). Dans ce tableau, les abréviations ont la signification suivante : MAPD = méthylacétylène + propadiène,

BBV = butadiène-1,2 + butyne-1 + vinylacétylène.

La coupe $C_4$ à traiter est d'abord mélangée au flux 6 de recyclage de l'effluent de la zone 3 (isomérisation squelettale), puis elle est soumise à un traitement d'hydrogénation et d'hydro-isomérisation dans la zone 1. Elle est introduite en continu, avec le débit massique indiqué dans le tableau 1, et sous une pression de 1,4 MPa, dans un premier réacteur comprenant un lit fixe d'un catalyseur constitué par du palladium sur alumine sulfuré au préalable. De l'hydrogène (mélangé à du méthane) est également injecté dans ce réacteur, comme indiqué dans le tableau 1 (flux 1+6). L'effluent de ce premier réacteur est ensuite traité dans un réacteur de finition chargé avec le même catalyseur. A la sortie (tableau 1, flux 3), la coupe est débarrassée des composés acétyléniques, et le butadiène a été transformé essentiellement en butènes, qui sont en majorité des butènes-2, le butène-1 ayant été isomérisé. La coupe est alors traitée dans une colonne de stabilisation, où l'hydrogène résiduel et le méthane sont séparés.

Dans la zone 2, la coupe $C_4$ hydro-isomérisée (effluent de la zone 1) est soumise à un fractionnement dans une colonne de distillation. Cette colonne comporte environ 90 plateaux et fonctionne à une pression de 0,7 MPa au ballon de reflux, de façon à permettre l'utilisation d'eau de réfrigération dans le condenseur de tête. Le taux de reflux est ajusté pour, d'une part, limiter la perte en butène-2 dans le distillat, et, d'autre part, réduire les teneurs en butène-1 et en isobutène dans le produit de fond, afin de limiter au maximum la formation des sous-produits pentènes et hexènes dans l'étape ultérieure de métathèse. Le flux 5 de tête de la distillation est dirigé vers la zone 3 d'isomérisation squelettale le flux 4 de pied de distillation est dirigé vers la zone 4 de métathèse. Ces deux flux ont la composition donnée au tableau 1.

Dans la zone 3, le catalyseur utilisé pour la réaction d'isomérisation squelettale de l'isobutène est composé d'alumine gamma renfermant 0,1 % en poids de titane. Il a été soumis à un traitement sous vapeur d'eau à 560 °C pendant 20 heures avec une pression partielle de vapeur d'eau égale à 0,08 MPa. Il est mis en oeuvre pour isomériser l'isobutène sortant de la zone 2 (flux 5) à une température de 500 °C, une pression de 0,1 MPa, un rapport molaire eau /isobutène égal à 2 et une vitesse spatiale de 1,3 $h^{-1}$. Dans ces conditions, la conversion de l'isobutène est de 57 % en poids et la sélectivité en n-butènes de 90 %. L'effluent de la zone 3 est séparé en un flux 6 de recyclage vers la zone 1 d'hydro-isomérisation, et en un flux 9 qui est une purge destinée à éviter l'accumulation de l'isobutane présent dans la coupe de charge. La composition de ces deux flux est donnée dans le tableau 1.

Dans la zone 4, la fraction de pied de distillation qui contient principalement du butène-2 (flux 4) est mise en réaction avec de l'éthylène (composition globale : flux 4+7 du tableau 1) sur un catalyseur de métathèse, constitué par de l'oxyde de rhénium sur alumine gamma (8 % en poids en rhénium métal), préparé selon les enseignements du brevet US-A-4 795 734. La coupe $C_4$ est mélangée à l'entrée du réacteur de métathèse avec de l'éthylène d'appoint, ainsi qu'avec des flux de recyclage d'éthylène et de butènes. Ce réacteur fonctionne en lit mobile, comme décrit dans le brevet FR-B-2 608 595, à une température de 35 °C et sous une pression de 3,5 MPa, et il est couplé avec un régénérateur fonctionnant à 550 °C sous pression atmosphérique. Le catalyseur est soutiré à intervalles de temps réguliers en bas du réacteur et transféré vers le régénérateur, d'où il est renvoyé en haut du réacteur, les transferts se faisant à travers des sas-tampons. En sortie de réacteur, l'éthylène non converti est fractionné dans une première colonne de distillation et recyclé. Une deuxième colonne de distillation sépare le propylène et les hydrocarbures en $C_4$ non convertis qui sont également recyclés. La composition de l'effluent de métathèse est indiquée dans le tableau 1, flux 8.

Le bilan global de la transformation s'établit donc comme suit. Pour 100 parties en poids (pp) de coupe $C_4$ sortie du vapocraqueur, on consomme 1,6 pp d'hydrogène et 44 pp d'éthylène, et on produit 118 pp de propylène. Au niveau du vapocraqueur d'où est issue la coupe $C_4$ traitée, ce bilan représente donc une consommation d'éthylène modeste, et une production supplémentaire de propylène importante.

L'avantage de ce procédé est donc de produire de façon très sélective un propylène de qualité polymérisation, grâce notamment à la métathèse d'une charge de butène-2 ne contenant que de faibles quantités de butène-1 et d'isobutène, charge obtenue par hydro-isomérisation et isomérisation squelettale d'une coupe $C_4$, ce qui permet de valoriser en propylène la totalité des oléfines de cette coupe.

## TABLEAU 1

| N° de flux (fig. 1) (kg/h) | 1 Charge C4 | 1 + 6 Entrée Hydro-Isomérisation | 3 Sortie Hydro-Isomérisation | 4 Sortie Stabilisation | 5 Tête colonne Isobutène | 6 Recyclage butènes | 9 Purge butènes | 4 Pied colonne Isobutène | 4 + 7 Entrée Métathèse | 8 Sortie Métathèse |
|---|---|---|---|---|---|---|---|---|---|---|
| (C3 + C3=) | 10 | 10 | 41 | | | | | | | |
| MAPD | 31 | 31 | | | | | | | | |
| Isobutane | 446 | 6424 | 6424 | 6424 | 6424 | 5978 | 446 | | | |
| n-Butane | 545 | 545 | 988 | 988 | | | | 988 | 988 | 988 |
| Isobutène | 5741 | 9588 | 9588 | 9588 | 9588 | 3847 | 287 | | | |
| Butène-1 | 3407 | 6455 | 1423 | 1423 | 1312 | 3048 | 238 | 111 | 111 | 89 |
| Butènes-2 | 2250 | 4990 | 18095 | 18095 | | 2740 | 198 | 18095 | 18095 | 1810 |
| Butadiène-1,3 | 8095 | 8095 | | | | | | | | |
| BBV | 104 | 104 | | | | | | | | |
| Hydrogène | | 343 | 26 | | | | | | | |
| Méthane | | 197 | 197 | | | | | | | |
| Ethylène | | | | | | | | | 9048 | 845 |
| Propylène | | | | | | | | | | 24428 |
| Lourds | | 504 | 504 | 504 | | 504 | 38 | 504 | 504 | 586 |
| **Total** | **20629** | **37286** | **37286** | **37022** | **17324** | **16117** | **1207** | **19698** | **28746** | **28746** |

EP 1 110 933 B1

**Revendications**

1.  Procédé de conversion en propylène d'une coupe $C_4$ oléfinique, ladite coupe comprenant des dioléfines, principalement du butadiène-1,3, du butène-1, du butène-2, de l'isobutène et des impuretés acétyléniques, ledit procédé comprenant les étapes suivantes, se déroulant successivement :

    1) l'hydrogénation sélective des dioléfines et des impuretés acétyléniques avec isomérisation du butène-1 en butènes-2, menée dans un réacteur, en présence d'un catalyseur, de façon à obtenir un effluent contenant majoritairement des butènes-2 et de l'isobutène, et ne contenant pratiquement pas de dioléfines ni de composés acétyléniques ;
    2) la séparation par distillation d'une coupe de tête contenant majoritairement l'isobutène et le butène-1 non converti dans la première étape, et d'une coupe de pied contenant essentiellement le butène-2 et le butane ; et
    4) la métathèse de la coupe butènes-2 issue de l'étape 2 avec de l'éthylène façon à obtenir un effluent contenant du propylène, la métathèse étant suivie d'une séparation du propylène ;

    ledit procédé comprenant en outre une étape 3 d'isomérisation squelettale de l'isobutène en n-butènes sur la fraction de tête de étape 2 de distillation, avec recyclage d'au moins une partie de l'effluent de ladite étape 3 vers l'étape 1, ladite étape 3 étant réalisée au moyen d'un catalyseur comprenant de l'alumine et du titane, à une température de 300 °C à 570 °C, une pression de 0,1 à 1 MPa, à une vitesse spatiale de 0,1 à 10 $h^{-1}$, et en présence d'injection d'eau, le rapport molaire eau injectée / hydrocarbures oléfiniques étant de 0,1 à 10.

2.  Procédé selon la revendication 1, **caractérisé en ce que** le catalyseur d'isomérisation squelettale utilisé dans l'étape 3 contient de l'alumine et de 0,03 à 0,6 % en poids de titane et de 0,05 à 5 % en poids d'un oxyde d'un élément du groupe IIIA.

3.  Procédé selon la revendication 1 ou 2, dans lequel l'étape 1 est effectuée par passage de ladite coupe en phase liquide sur un catalyseur comprenant au moins un métal choisi dans le groupe formé par le nickel, le palladium et le platine, déposé sur un support, à une température de 0 à 200 °C, une pression de 0,1 à 5 MPa, une vitesse spatiale de 0,5 à 10 h $^{-1}$, avec un rapport molaire $H_2$ / dioléfines de 0,5 à 5.

4.  Procédé selon la revendication 1 à 3, dans lequel le catalyseur de l'étape 1 contient de 0,05 à 10 % en poids de soufre.

5.  Procédé selon l'une des revendications 1 à 4, dans lequel le catalyseur de l'étape 1 a été traité, avant son chargement dans le réacteur d'hydrogénation, par au moins un composé soufré dilué dans un solvant, et que le catalyseur obtenu, contenant de 0,05 à 10 % en poids de soufre est chargé dans le réacteur et activé sous atmosphère neutre ou réductrice à une température de 20 à 300 °C, une pression de 0,1 à 5 MPa et une VVH de 50 à 600 $h^{-1}$, et que la charge est mise au contact dudit catalyseur activé.

6.  Procédé selon l'une des revendications 3 à 5, dans lequel le catalyseur de l'étape 1 est constitué par du palladium déposé sur de l'alumine, et du soufre.

7.  Procédé selon l'une des revendications 1 à 6, dans lequel l'isomérisation du butène-1 en butène-2 réalisée dans l'étape 1 et la distillation de l'étape 2 sont réunies en une seule étape faisant intervenir une colonne de distillation réactive incluant intérieurement ou extérieurement un catalyseur d'isomérisation tel que décrit pour l'étape 1.

8.  Procédé selon l'une des revendications 1 à 7 dans lequel la métathèse est réalisée dans l'étape 4 en présence d'un catalyseur comprenant au moins un oxyde de rhénium déposé sur un support, à une température de 0 à 200 °C, et à une pression au moins égale à la tension de vapeur du mélange réactionnel à la température de réaction.

9.  Procédé selon la revendication 8, dans lequel ledit catalyseur contient de l'oxyde de rhénium à raison de 0,01 à 20 % en poids exprimé en rhénium métallique, déposé sur un support contenant au moins 75 % en poids d'alumine et 0,01 à 30 % en poids d'au moins un oxyde d'un métal choisi dans le groupe formé par le niobium et le tantale.

10. Procédé selon l'une des revendications 8 ou 9, dans lequel la métathèse est effectuée avec un catalyseur en lit mobile.

11. Procédé selon l'une des revendications 1 à 11, dans lequel la coupe $C_4$ à traiter est une coupe de vapocraquage et l'éthylène utilisé dans l'étape de métathèse provient de l'opération de vapocraquage.

12. Procédé selon l'une des revendications 1 à 12, dans lequel la coupe de pied de l'étape 2 de distillation contient au plus 1 % en poids d'isobutène et au plus 1 % en poids de butène-1.

13. Installation pour la conversion d'une coupe $C_4$ oléfinique en isobutène et en propylène, comportant successivement :

> 1) une zone 1 d'hydrogénation sélective avec isomérisation du butène-1 en butène-2, ladite zone comportant au moins un moyen 1 pour l'introduction de la coupe C4 à convertir, au moins un moyen 3 pour la sortie de l'effluent et au moins un moyen 2 pour l'introduction de l'hydrogène, ladite zone comportant également au moins un lit de catalyseur ;
> 2) une zone 2 de séparation, comportant au moins un moyen 3 pour l'introduction de l'effluent issu de la zone 1, au moins un moyen 5 pour la sortie de l'isobutène et du butène-1, au moins un moyen 4 pour la sortie du butène-2 et du n-butane ;et
> 4) une zone 4 de métathèse contenant au moins un lit de catalyseur et comprenant au moins un moyen 4 d'introduction de l'effluent issu de la zone 2, au moins un moyen 7 d'introduction de l'éthylène et au moins un moyen 8 pour la sortie du propylène.

ladite installation comprenant en outre une zone 3 d'isomérisation squelettale, comportant au moins un moyen 5 pour l'introduction de l'effluent issu de la zone 2, au moins un moyen 6 pour le recyclage de la sortie de la zone 3 à l'entrée de la zone 1 et au moins un moyen 9 pour purger l'isobutane éventuellement présent, ladite zone comportant également au moins un lit de catalyseur comprenant de préférence de l'alumine et du titane.

14. Installation selon la revendication 13, dans laquelle la zone de métathèse contient un lit mobile de catalyseur.

15. Installation selon l'une des revendications 13 et 14, dans laquelle le moyen d'introduction de la coupe $C_4$ à convertir est relié à une zone de vapocraquage, et en ce que le moyen d'introduction de l'éthylène dans la zone de métathèse est relié à ladite zone de vapocraquage.

**Claims**

1. Process for converting into propylene an olefinic C4 fraction, whereby said fraction comprises diolefins, primarily butadiene-1,3, butene-1, butene-2, isobutene and acetylenic impurities, and whereby said process comprises the following stages that take place successively:

> 1) the selective hydrogenation of diolefins and acetylenic impurities with isomerization of butene-1 into butenes-2, carried out in a reactor, in the presence of a catalyst, in order to obtain an effluent that contains for the most part butenes-2 and isobutene, and that contains virtually no diolefins or acetylenic compounds;
> 2) the separation by distillation of a top fraction that contains for the most part isobutene and unconverted butene-1 in the first stage, and a bottom fraction that contains essentially butene-2 and butane; and
> 4) the metathesis of the butenes-2 fraction that is obtained from stage 2 with the ethylene so as to obtain an effluent that contains propylene, whereby the metathesis is followed by a separation of the propylene;

whereby said process also comprises a stage 3 of skeletal isomerization of the isobutene into n-butenes in the top fraction of stage 2 of distillation, with recycling of at least a portion of the effluent of stage 3 to stage 1, said stage 3 being carried out with the use of a catalyst comprising alumina and titanium at a temperature of 300° to 570°C, under a pressure of 0.1 to 1 MPa, at a space velocity of 0.1 to 10 h-1 and in the presence of water injection, whereby the molar ratio injected water/olefinic hydrocarbons is from 0.1 to 10.

2. A process according to claim 1, **characterized in that** the catalyst for skelettal isomerization used in stage 3 contains alumina and from 0.03to 0.6% by weight of titanium and from 0.05 to 5% by weight of an oxide of an element of group IIIA.

3. Process according to claim 1 or 2, wherein stage 1 is carried out by running said fraction in the liquid phase over a catalyst that comprises at least one metal that is selected from the group that is formed by nickel, palladium and platinum, deposited on a substrate, at a temperature of 0 to 200°C, a pressure of 0.1 to 5 MPa, a volumetric flow rate of 0.5 to 10 h-1, with an H2/diolefin molar ratio of 0.5 to 5.

4. Process according to claim 1 to 3, wherein the catalyst of stage 1 contains 0.05 to 10% by weight of sulfur.

5. Process according to one of claims 1 to 4, wherein the catalyst of stage 1 was treated, before being loaded into the hydrogenation reactor, by at least one sulfur-containing compound that is diluted in a solvent, and wherein the catalyst that is obtained and that contains 0.05 to 10% by weight of sulfur is loaded into a reactor and activated under a neutral atmosphere or a reducing atmosphere at a temperature of 20 to 300°C, a pressure of 0.1 to 5 MPa and a VVH of 50 to 600 h-1, and wherein the feedstock is brought into contact with said activated catalyst.

6. Process according to one of claims 3 to 5, wherein the catalyst of stage 1 consists of palladium that is deposited on alumina and sulfur.

7. Process according to one of claims 1 to 6, wherein the isomerization of butene-1 into butene-2 that is carried out in stage 1 and the distillation of stage 2 are joined in a single stage that causes a reactive distillation column that includes on the inside or outside an isomerization catalyst as described for stage 1 to take effect.

8. Process according to one of claims 1 to 7, wherein the metathesis is carried out in stage 4 in the presence of a catalyst that comprises at least one rhenium oxide that is deposited on a substrate at a temperature of 0 to 200°C, and at a pressure that is at least equal to the vapor pressure of the reaction mixture at the reaction temperature.

9. Process according to claim 8, wherein said catalyst contains rhenium oxide at a rate of 0.01 to 20% by weight expressed in metallic rhenium, deposited on a substrate that contains at least 75% by weight of alumina and 0.01 to 30% by weight of at least one oxide of a metal that is selected from the group that is formed by niobium and tantalum.

10. Process according to one of claims 8 or 9, wherein the metathesis is carried out with a moving-bed catalyst.

11. Process according to one of claims 1 to 11, wherein the C4 fraction that is to be treated is a steam-cracking fraction, and the ethylene that is used in the metathesis stage is obtained from the steam-cracking operation.

12. Process according to one of claims 1 to 12, wherein the bottom fraction of distillation stage 2 contains at most 1% by weight of isobutene and at most 1% by weight of butene-1.

13. Installation for the conversion of an olefinic C4 fraction into isobutene and into propylene, successively comprising:

1) a selective hydrogenation zone 1 with isomerization of butene-1 into butene-2, whereby said zone comprises at least one means 1 for introducing the C4 fraction that is to be converted, at least one means 3 for the output of the effluent and at least one means 2 for the introduction of hydrogen, whereby said zone also comprises at least one catalyst bed;
2) a zone 2 for separation that comprises at least one means 3 for introducing the effluent that is obtained from zone I, at least one means 5 for the output of isobutene and butene-1, at least one means 4 for the output of butene-2 and n-butane; and
4) a metathesis zone 4 that contains at least one catalyst bed and that comprises at least one means 4 for introducing the effluent that is obtained from zone 2, at least one means 7 for introducing ethylene and at least one means 8 for the output of propylene,

whereby said installation also comprises a skeletal-isomerization zone 3 that comprises at least one means 5 for introducing the effluent that is obtained from zone 2, at least one means 6 for recycling from the outlet of zone 3 to the inlet of zone 1 and at least one means 9 for purging the optionally present isobutane, whereby said zone also comprises at least one catalyst bed that preferably comprises alumina and titanium.

14. Installation according to claim 13, wherein the metathesis zone contains a catalyst moving bed.

15. Installation according to one of claims 13 and 14, wherein the means for introducing the C4 fraction that is to be converted is connected to a steam-cracking zone and wherein the means for introducing ethylene into the metathesis zone is connected to said steam-cracking zone.

**Patentansprüche**

1. Verfahren zur Umwandlung einer olefinischen C$_4$-Fraktion zu Propylen, welche Fraktion Diolefine, hauptsächlich 1,3-Butadien, 1-Buten, 2-Buten, Isobuten und acetylenische Verunreinigungen umfasst, wobei das Verfahren die folgenden aufeinanderfolgend ablaufenden Stufen umfasst:

   1) die selektive Hydrierung der Diolefine und der acetylenischen Verunreinigungen mit Isomerisierung des 1-Butens zu 2-Butenen, die in einem Reaktor in Gegenwart eines Katalysators derart geführt wird, dass ein Abstrom erhalten wird, der mehrheitlich 2-Butene und Isobuten enthält und praktisch weder Diolefine noch acetylenische Verbindungen enthält;

   2) die Trennung einer Kopffraktion durch Destillation, welche mehrheitlich Isobuten und nicht in der ersten Stufe umgewandeltes 1-Buten enthält, und einer Bodenfraktion, die im wesentlichen 2-Buten und Butan enthält; und

   3) die Metathesis der 2-Butenfraktion aus der Stufe 2 mit Ethylen derart, dass ein propylenhaltiger Abstrom erhalten wird, wobei die Metathesis von einer Trennung des Propylens gefolgt ist;

   wobei das Verfahren außerdem eine Stufe 3 zur Gerüstisomerisierung des Isobutens zu n-Butenen auf der Kopffraktion der Destillationsstufe 2 mit Rezyklierung wenigstens eines Teils des Abstroms der Stufe 3 zur Stufe 1 hin umfasst, wobei die Stufe 3 mit einem Katalysator, der Aluminiumoxid und Titan umfasst, bei einer Temperatur von 300°C bis 570°C, einem Druck von 0,1 bis 1 MPa, einer Raumgeschwindigkeit von 0,1 bis 10 h$^{-1}$ und in Gegenwart einer Wassereinspritzung durchgeführt wird, wobei das molare Verhältnis eingespritztes Wasser / olefinische Kohlenwasserstoffe 0,1 bis 10 ist.

2. Verfahren nach Anspruch 1, **dadurch gekennzeichnet, dass** der in Stufe 3 verwendete Katalysator zur Gerüstisomerisierung Aluminiumoxid und 0,03 bis 0,6 Gew.-% Titan und 0,05 bis 5 Gew.-% eines Oxids eines Elements der Gruppe IIIA enthält.

3. Verfahren nach Anspruch 1 oder 2, bei dem Stufe 1 durch Durchlauf der Flüssigphasenfraktion über einen Katalysator durchgeführt wird, der wenigstens ein Metall umfasst, das aus der Gruppe gewählt ist, die gebildet wird durch Nickel, Palladium und Platin, abgeschieden auf einen Träger bei einer Temperatur von 0 bis 200°C, einem Druck von 0,1 bis 5 MPa, einer Raumgeschwindigkeit von 0,5 bis 10 h$^{-1}$ mit einem molaren Verhältnis H$_2$ / Diolefine von 0,5 bis 5.

4. Verfahren nach Anspruch 1 bis 3, bei dem der Katalysator der Stufe 1 0,05 bis 10 Gew.-% Schwefel enthält.

5. Verfahren nach einem der Ansprüche 1 bis 4, bei dem der Katalysator der Stufe 1 vor seinem Beladen in den Hydrierreaktor durch wenigstens eine in einem Lösungsmittel verdünnte Schwefelverbindung behandelt worden ist und so, dass der erhaltene, 0,05 bis 10 Gew.-% Schwefel enthaltende Katalysator in den Reaktor geladen und unter neutraler der reduzierender Atmosphäre bei einer Temperatur von 20 bis 300°C, einem Druck von 0,1 bis 5 MPa und einer VVH von 50 bis 600 h$^{-1}$ aktiviert wird und dass die Beschickung mit dem aktivierten Katalysator kontaktiert wird.

6. Verfahren nach einem der Ansprüche 3 bis 5, bei dem der Katalysator der Stufe 1 aus Palladium, abgeschieden auf Aluminiumoxid und Schwefel besteht.

7. Verfahren nach einem der Ansprüche 1 bis 6, bei dem die in der Stufe 1 durchgeführte Isomerisierung des 1-Butens zu 2-Buten und die Destillationsstufe 2 in einer einzigen Stufe vereint sind, welche eine reaktive Destillationskolonne einwirken lässt, die innen oder außen einen wie für Stufe 1 beschriebenen Isomerisierungskatalysator einschließt.

8. Verfahren nach einem der Ansprüche 1 bis 7, bei dem die Metathesis in der Stufe 4 in Gegenwart eines Katalysators durchgeführt wird, der wenigstens ein Rheniumoxid umfasst, das auf einen Träger bei einer Temperatur von 0 bis 200°C und einem Druck wenigstens gleich der Dampfspannung des Reaktionsgemischs bei der Reaktionstemperatur abgeschieden ist.

9. Verfahren nach Anspruch 8, bei dem der Katalysator Rheniumoxid im Verhältnis von 0,01 bis 20 Gew.-%, ausgedrückt an metallischem Rhenium umfasst, das auf einem Träger abgeschieden ist, der wenigstens 75 Gew.-% Aluminiumoxid und 0,01 bis 30 Gew.-% wenigstens eines Oxids eines Metalls enthält, das aus der Gruppe gewählt ist, die gebildet wird durch Niob und Tantal.

10. Verfahren nach einem der Ansprüche 8 oder 9, bei dem die Metathesis mit einem Katalysator im beweglichen Bett durchgeführt wird.

11. Verfahren nach einem der Ansprüche 1 bis 11, bei dem die zu behandelnde $C_4$-Fraktion eine Dampfcrackfraktion ist und das in der Metathesisstufe verwendete Ethylen von einem Dampfcrackvorgang kommt.

12. Verfahren nach einem der Ansprüche 1 bis 12, bei dem die Bodenfraktion der Destillationsstufe 2 höchstens 1 Gew.-% Isobuten und höchstens 1 Gew.-% 1-Buten enthält.

13. Anlage für die Umwandlung einer olefinischen $C_4$-Fraktion zu Isobuten und zu Propylen, aufeinanderfolgend umfassend:

1) eine Zone 1 zur selektiven Hydrierung mit Isomerisierung des 1-Butens zu 2-Buten, wobei die Zone wenigstens ein Mittel 1 zur Einführung der umzuwandelnden $C_4$-Fraktion, wenigstens ein Mittel 3 zum Austritt des Abstroms und wenigstens ein Mittel 2 zur Einführung von Wasserstoff umfasst, wobei die Zone auch wenigstens ein Katalysatorbett umfasst;

2) eine Zone 2 zur Trennung, die wenigstens ein Mittel 3 zur Einführung des Abstroms aus der Zone 1, wenigstens ein Mittel 5 zum Austritt des Isobutens und des 1-Butens, wenigstens ein Mittel 4 zum Austritt des 2-Butens und n-Butans umfasst; und

4) eine Zone 4 zur Metathesis, die wenigstens ein Katalysatorbett enthält und wenigstens ein Mittel 4 zur Einführung des Abstroms aus der Zone 2, wenigstens ein Mittel 7 zur Einführung des Ethylens und wenigstens ein Mittel 8 zum Austritt des Propylens umfasst;

wobei die Anlage außerdem eine Zone 3 zur Gerüstisomerisierung umfasst, die wenigstens ein Mittel 5 zur Einführung des Abstroms aus der Zone 2, wenigstens ein Mittel 6 zum Rezyklieren vom Ausgang der Zone 3 zum Eingang der Zone 1 und wenigstens ein Mittel 9 zum Abführen des gegebenenfalls vorliegenden Isobutans umfasst, wobei die Zone auch wenigstens ein Katalysatorbett umfasst, das vorzugsweise Aluminiumoxid und Titan umfasst.

14. Analge nach Anspruch 13, bei der die Metathesiszone ein bewegliches Katalysatorbett enthält.

15. Analge nach einem der Ansprüche 13 und 14, bei der das Mittel zur Einführung der umzuwandelnden $C_4$-Fraktion mit einer Dampfcrackzone verbunden ist und so, dass das Mittel zur Einführung von Ethylen in die Metathesiszone mit der Dampfcrackzone verbunden ist.

FIG. 1

EP 1 110 933 B1